# EUROPEAN PATENT APPLICATION

(11) **EP 4 497 455 A1**
(43) Date of publication of application: **29.01.2025**
(21) Application number: 23187179.9
(22) Date of filing: 24.07.2023
(51) Int. Cl.: A61M 1/36, A61M 39/10

(54) **TRANSDUCER PROTECTOR FOR AN EXTRACORPOREAL CIRCUIT**

(71) Applicant: Fresenius Medical Care Deutschland GmbH, 61352 Bad Homburg (DE)
(72) Inventor: CAVALLI, Riccardo, 61352 Bad Homburg, (DE); VENERONI, Alain, 61352 Bad Homburg, (DE); BRONZATO, Luca, 61352 Bad Homburg, (DE)
(74) Representative: Behr, Wolfgang

(57) **Abstract**

The present invention comprises a transducer protector for protecting a pressure transducer connected to an extracorporeal circuit, comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end. The transducer protector is characterized in that the second connector is a snap-fit connector.

## Description

The present invention relates to a transducer protector for protecting a pressure transducer connected to an extracorporeal blood circuit.

In medical treatments which require an extracorporeal circuit, in particular in blood-treatments such as hemodialysis, the pressure in the extracorporeal circuit, for example the arterial and/or the venous pressure, must be constantly monitored. This is usually achieved by means of a pressure transducer connected to the main circuit of the extracorporeal circuit by a branch pipe.

In order to protect the pressure transducer, which is typically located inside a dialysis machine, from any contact with the patient's blood, a transducer protector is usually provided between the pipe and the pressure transducer. In particular, the transducer protector may be provided as a part of the extracorporeal circuit as a disposable, and during treatment being connected to a pressure transducer interface.

Document EP 2 226 087 A1 shows a well-established configuration of a transducer protector for an extracorporeal circuit, comprising a main body comprising a fluid path, a first connector for fluidly coupling a first end of the fluid path to a pipe of the extracorporeal circuit, a second connector for fluidly coupling a second end of the fluid path to a pressure transducer interface, and a hydrophobic gas-permeable membrane arranged in the fluid path for separating the first end of the fluid path from the second end.

In this known transducer protector, the second connector is configured as a female type Luer-type connector, comprising a screw section for screwing the second connector onto a corresponding screw section of the pressure transducer interface. Further, the main body of the transducer protector is formed by means of two plastic half-shells enclosing between them a hydrophobic membrane, the two sections forming a circular section arranged between the two tubular connectors. The circular section enclosing the membrane is used as a gripping section for turning the transducer protector for closing and opening the Luer connection.

This transducer protector provides a robust design used for many years in the market. However, it has non-optimal usability and is in particular difficult to handle due to the reduced surface of the circular central element. Further, due to the rotating movement needed to screw the component on the Luer lock male connector, fixation is difficult. Finally, the user is not aware if the transducer protector is properly connected or not, and therefore not aware if the connection is tight. Further, manufacture of the transducer protector is difficult.

Despite these disadvantages, the standard configuration using two half shells and a Luer lock connection has nevertheless been the generally accepted solution for connecting a transducer protector to the corresponding pressure transducer interface in the market. So far, most efforts in improving known transducer protectors have centered on the improvement of the safety of the separation function of the membrane. For example, document EP 2 408 490 B1 shows a transducer protector using two membranes arranged in series.

The object of the present invention is to provide an improved transducer protector for an extracorporeal circuit and a corresponding pressure transducer interface. In particular, the present invention sets out to avoid at least some of the disadvantages of the known solution described above.

This object is solved by a transducer protector according to claims 1 and 8 as well as a pressure transducer interface according to claim 10.

In a first aspect, the present application comprises a transducer protector for protecting a pressure transducer connected to an extracorporeal circuit, the transducer protector comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end. In particular, the first connector and the second connector are arranged at the ends of the fluid path extending through the main body of the transducer protector, with the hydrophobic gas-permeable membrane being arranged in the fluid path such that the pipe of the extracorporeal circuit and the pressure transducer interface, when coupled with the transducer protector, are fluidly connected to each other through the hydrophobic gas-permeable membrane. According to the first aspect of the present invention, the second connector is a snap-fit connector.

The inventors of the present invention have realized that a snap-fit connector is decisively easier to handle than the Luer connectors used in the prior art. Further, the user is provided with a tactile and/or audible feedback, such that proper connection is ensured. A snap-fit connector further allows for additional advantages as described in the following.

In an embodiment of the present invention, the second connector is configured to be snap-fit engaged with the pressure transducer interface with a single axial movement in the direction of the fluid path at its second end.

Because the snap-fit connection provided by the second connector is engaged with a single axial movement in the direction of the fluid path at its second end, establishing the connection with the pressure transducer interface is simpler than in prior art solutions requiring a rotating movement. Thereby, the usability is improved.

In an embodiment of the present invention, the first connector is a tubular element configured for receiving an end section of the pipe of the extracorporeal circuit.

In an embodiment of the present invention, the end section of the pipe is glued into the tubular element.

In an embodiment of the present invention, the first connector comprises a receiving section for a third connector. The third connector may, e.g., be a male or female Luer connector, a bayonet connector or screw-type connector.

In an embodiment of the present invention, the second connector comprises at least one snap-fit element of the cantilever type.

In an embodiment of the present invention, the snap-fit element is a snap-fit arm connected to a main body of the transducer protector and having a free end comprising a snap-fit geometry for snap engaging a counter element of the pressure transducer interface.

In an embodiment of the present invention, the snap-fit geometry comprises a hook section for snap engaging a hook section of the counter element of the pressure transducer interface.

In an embodiment of the present invention, the snap-fit arm comprises a chamfer section arranged at its free end, the hook section arranged behind the chamfer section.

The chamfer section may be configured for contacting a front side of the counter element of the pressure transducer interface, such contact deforming the snap-fit arm when the snap-fit arm is moved in an axial direction until the hook section of the snap-arm engages with the hook section on the pressure transducer interface.

In an embodiment of the present invention, the snap-fit arm extends from a connection point where it is connected to the main body of the transducer protector in a direction along of the fluid path of the transducer protector towards the pressure transducer interface. In particular, the snap-fit arm may extend with an angle of less than 20° with respect to an axial direction defined by the direction of the fluid path at its second end.

In a first variant, the snap-fit arm extends from the connection point only in the direction towards the pressure transducer interface. Thereby, the handling area can be increased.

In a second variant, the snap-fit arm comprises a first section extending from the connection point in a direction away from the pressure transducer interface and a second section extending from the connection point in a direction towards the pressure transducer interface, each section having a fee end. The first section extending from the connection point in a direction away from the pressure transducer interface may form a handling area for gripping the transducer protector.

In an embodiment of the present invention, the snap-fit connection provided by the second connector is a releasable connection.

In an embodiment of the present invention, the second connector is configured such that the snap-fit connection is releasable by flexing at least one snap-fit element by applying pressure to a handling section of the snap-fit element. This ensures that the snap-fit connection can be reliably disengaged.

In an embodiment of the present invention, the snap-fit element is configured to be releasable by a pressure acting on the handling section of the snap-fit element from an outer side in a direction towards the main body of the connector.

In an embodiment of the present invention, the snap-fit element is a snap-fit arm as defined herein, and in particular a snap-fit arm as described above.

In an embodiment of the present invention, the second connector comprises at least two separate snap-fit elements. The snap-fit elements may each be configured as described above for the at least one snap-fit element.

In an embodiment of the present invention, the snap-fit elements are arranged on opposite sides of an axis defined by the fluid path at its second end.

In an embodiment of the present invention, the snap-fit connection provided by the second connector is releasable by pressing the handling sections of the snap-fit elements from an outer side in a direction towards each other. This allows easy operation of the transducer protector.

In an embodiment of the present invention, the snap-fit elements each are a snap-fit arm as described herein.

In an embodiment of the present invention, the second end of the fluid path is configured as a tubular element to be sealingly engaged with a fluid path of the pressure transducer interface, wherein preferably a snap-fit element or snap-fit elements of the second connector extend from a connection point with the main body of the transducer protector towards the pressure transducer interface on an outside and with a distance to the tubular element, the snap-fit element or snap-fit elements being deformable towards and/or away from the tubular element. This allows a reliable sealing provided by the tubular element combined with a reliable snap-fit connection provided by the snap-fit element or snap-fit elements without any interference of the one with the other.

In a variant, the second end of the fluid path is surrounded by a flange surface laminated with a sealing layer for sealingly engaging the pressure transducer interface, wherein preferably, the hydrophobic gas-permeable membrane is bonded to the sealing layer.

The bonding of the hydrophobic gas-permeable membrane to the body of the transducer protector and/or the sealing layer is preferably done by heat bonding, ultrasonic welding or gluing. The preferred method for bonding the membrane to the sealing layer is ultrasonic welding.

In an embodiment of the present invention, a snap-fit geometry of the second connector is configured to snap engage a counter element of the pressure transducer interface from an inner side. In particular, the snap-fit geometry may therefore be provided on the snap-fit element or elements of the transducer protector on an outer side.

In an embodiment of the present invention, a handling section of at least one snap-fit element of the second transducer is arranged between a connection point where the snap-fit element is connected to the main body of the transducer protector and a free end of the snap-fit element configured for connecting to the pressure transducer interface. Thereby, a particularly large handling section can be provided.

In a second variant, a snap-fit geometry of the second connector is configured to snap engage a counter element of the pressure transducer interface from an outer side.

In an embodiment of the present invention, a handling section of at least one snap-fit element of the second transducer is arranged between a connection point where the snap-fit element is connected to the main body of the transducer protector and a free end of a first section of the snap-fit element extending in a direction away from the pressure transducer interface.

In an embodiment of the present invention, the second connector comprises at least one snap-fit arm extending in a direction along of the fluid path of the transducer protector towards the pressure transducer interface, wherein the length of the snap-fit arm in a direction defined by the fluid path is at least 50% of the length of the fluid path, preferably at least 65%, further preferably at least 80%. This simplifies the handling of the transducer protector.

In an embodiment of the present invention, a handling section of the snap-fit element is provided with ridges, the ridges preferably extending in a direction of the fluid path.

In an embodiment of the present invention, a snap-fit element or a snap-fit geometry of the second connector has a rounded shape, preferably an essentially circular shape with respect to an axis defined by the fluid path at its second end. This allows for a particularly efficient connection. In particular, the shape may be circular.

In an embodiment of the present invention, in a cross section, an outer and/or an inner edge of the snap-fit element or a snap-fit geometry of the second connector has a rounded shape, preferably an essentially circular shape. In particular, the shape may be circular.

In a first variant, the transducer protector is formed by two molded parts, each part comprising one of the first and the second connector, and/or wherein the hydrophobic gas-permeable membrane is arranged between the two molded parts.

In a second variant of the present invention, the transducer protector is formed by an integrally molded part comprising the entire fluid path and preferably both the first and the second connector. This reduces the production costs.

In a third variant of the present invention, which may be combined with the second variant, the hydrophobic gas-permeable membrane is bonded to a main body of the transducer protector from the side of the second end of the fluid path. In particular, the hydrophobic gas-permeable membrane is bonded to a step portion provided within the fluid path of the transducer protector facing the second end.

The second and third variant are the subject matter of the present also independently of the snap-fit connection according to the first aspect, and in particular form a second and third independent aspect as described in the following.

In a second aspect, the present application comprises a transducer protector for an extracorporeal circuit, the transducer protector comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end. In particular, the first connector and the second connector are arranged at the ends of the fluid path extending through the main body of the transducer protector, with the hydrophobic gas-permeable membrane being arranged in the fluid path such that the pipe of the extracorporeal circuit and the pressure transducer interface, when coupled with the transducer protector, are fluidly connected to each other through the hydrophobic gas-permeable membrane. According to the second aspect of the present invention, the transducer protector comprises an integrally molded part comprising the entire fluid path. This simplifies manufacture.

In an embodiment of the present invention, the integrally molded part comprises the entire fluid path and at least the sealing sections of the first and the second connector configured for sealingly connecting to the pipe and the pressure transducer interface.

In an embodiment of the present invention, the integrally molded part comprises the entire fluid path, with the second end of the fluid path being formed by a tubular element configured for sealingly connecting to the pressure transducer interface, and in particular to a sealing element of the pressure transducer interface.

In first variant of the second aspect of the present invention, mechanical locking elements, in particular snap-fit elements, of the second connector may be provided as a separate part connected to the integrally molded part.

In second, preferred variant of the second aspect of the present invention, the integrally molded part comprises the first and the second connector, i.e. all the parts of the first and the second connector.

In an embodiment of the present invention, the integrally molded part transducer is the only structural part of protector.

In an embodiment of the present invention, the transducer protector is formed by the integrally molded part and the hydrophobic gas-permeable membrane, and optionally one or more sealing elements, with no further parts attached to transducer protector.

In a second aspect, the present application comprises a transducer protector for protecting a pressure transducer connected to an extracorporeal circuit, the transducer protector comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end. In particular, the first connector and the second connector are arranged at the ends of the fluid path extending through the main body of the transducer protector, with the hydrophobic gas-permeable membrane being arranged in the fluid path such that the pipe of the extracorporeal circuit and the pressure transducer interface, when coupled with the transducer protector, are fluidly connected to each other through the hydrophobic gas-permeable membrane. According to the third aspect of the present invention, the hydrophobic gas-permeable membrane is bonded to a step portion of the transducer protector, the bonding area of the hydrophobic gas-permeable membrane being accessible from the second end of the fluid path.

As in the second aspect, this simplifies manufacture of the transducer protector.

In an embodiment of the third aspect of the present invention, the step portion is provided within the fluid path or at the second end of the fluid path.

In an embodiment of the present invention, the inner diameter of the fluid path between its second end and the step portion is larger than the inner diameter of the fluid path at the step portion.

In an embodiment of the present invention, the inner diameter and/or the cross-section of the fluid path between its second end and the hydrophobic gas-permeable membrane is larger than the outer diameter and/or outer shape of the hydrophobic gas-permeable membrane.

This simplifies the mounting of the hydrophobic gas-permeable membrane to the step portion, because the membrane and/or a bonding tool can be inserted into the transducer protector through the second end for manufacturing the transducer protector.

In an embodiment of the present invention, the second end of the fluid path is formed by a tubular element configured for sealingly connecting to the pressure transducer interface, an inner diameter of the tubular element being larger than the inner diameter of a tubular element forming the first connector. Preferably, the inner diameter of the tubular element forming the second end of the fluid path is at least 120 % of the inner diameter of a tubular element forming the first connector, preferably at least 150 %. These features are preferred for transducer protectors according to all aspects of the present invention.

In an embodiment of the present invention, the inner diameter and/or the inner cross-section of the step portion is larger than the inner diameter and/or the cross-section of the first connector and/or the pipe connected thereto. This provides an effective surface of the hydrophobic gas-permeable membrane that is larger than the cross-section of the first connector and/or the pipe connected thereto.

The second and the third aspect of the present invention are preferably combined.

Further, the first and the second aspect and/or the first and the third aspect are preferably combined.

Further preferably, the first, second and third aspect are combined.

The present invention further comprises a pressure transducer interface configured for connecting to the second connector of a transducer protector as defined above, the pressure transducer interface comprising a fluid path coupled to a pressure transducer and a snap-fit counter element for engaging with a snap-fit geometry of a snap-fit element of the second connector. The pressure transducer interface provides the same advantages as described above for the transducer protector according to the first aspect. The pressure transducer interface may comprise the pressure transducer.

In an embodiment of the present invention, the pressure transducer interface comprises a sealing element for sealingly engaging a tubular section of the second connector of the transducer protector. The sealing element may contact a front surface of the tubular element and/or an inner or outer surface of the tubular element. The sealing element may be provided by a sealing ring or a conical section.

In an embodiment of the present invention, the sealing element engages with a front surface of the tubular section or a side surface of the tubular section.

In an embodiment of the present invention, the sealing element is formed by a cone section engaging with an inner cone section of the tubular section, in particular with a Luer cone section.

In an embodiment of the present invention, the snap-fit counter element is formed by a bracket having a middle section for engaging with the snap-fit geometry of the snap-fit element of the second connector, the bracket freely extending between two connection sections where it is connected to a main surface of the pressure transducer interface. The bracket may in particular project from the main surface towards the transducer protector.

The brackets allow a user to access from below the bracket to easily clean/disinfect around the fluid path of the pressure transducer interface.

In an embodiment of the present invention, the main surface is an essentially flat surface from which the bracket projects in a direction towards the transducer protector.

In a variant, the counter element is formed by an undercut of a recess provided in a main surface of the pressure transducer interface.

The pressure transducer interface and/or the transducer protector may have a feature to prevent the improper mounting, in particular to avoid a mounting in a position that is rotated by 90° around its axis.

In an embodiment of the present invention, the pressure transducer interface comprises, in addition to the snap-fit counter element, a threaded section configured for engaging with another type of transducer protector comprising a Luer connector.

Such a pressure transducer interface is compatible both with prior art transducer protectors and with the inventive transducer protector.

The present invention further comprises a tubing system comprising a transducer protector as defined herein and at least one component of an extracorporeal blood circuit.

In particular, the component of the extracorporeal blood circuit is connected to the first connector via a tube, preferably glued to or inserted into a section of the first connector.

In an embodiment, the component of the extracorporeal blood circuit is connected to the first connector via a third connector. The third connector may, e.g., be a male or female Luer connector, a bayonet connector or screw-type connector.

Preferably, the tube is a tube leading from a venous chamber of the extracorporeal blood circuit for conveying air across the hydrophobic gas-permeable membrane.

The component of the extracorporeal blood circuit may in particular be a venous chamber.

The tubing system may comprise further components of an extracorporeal blood circuit, such as a venous and/or an arterial line or connection lines connectable to a venous and/or an arterial line.

The present invention further comprises a system comprising a transducer protector as defined herein and a pressure transducer interface as defined herein.

The present invention further comprises a blood treatment machine, in particular a dialysis machine, comprising a pressure transducer interface as defined herein.

In particular, the pressure transducer interface may be provided on a casing of the blood treatment machine. The main surface of the pressure transducer interface may be provided by an outer surface of the casing.

The present invention further comprises a blood treatment machine, in particular a dialysis machine, comprising a pressure transducer interface and a transducer protector as defined herein. The blood treatment machine may further comprise an extracorporeal blood circuit.

The present invention further comprises the use of a transducer protector according to the first aspect for connecting a pipe of an extracorporeal circuit to a pressure transducer by snap-fit connecting a snap-fit element of the second connector of the transducer protector to a snap-fit counter element of a pressure transducer interface, in particular to a pressure transducer interface of a blood treatment machine, in particular of a blood treatment machine as described above.

In a further independent aspect, the present invention comprises a method for manufacturing a transducer protector for an extracorporeal circuit, the transducer protector comprising a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic membrane arranged in the fluid path for separating the first end of the fluid path from the second end; the method comprising the step of:
- integrally molding the main body of the protector comprising the entire fluid path and preferably both the first and the second connector,
   and/or
- bonding the hydrophobic gas-permeable membrane to a step portion of the transducer protector by accessing the bonding area of the hydrophobic gas-permeable membrane from and preferably through the second end of the fluid path, the step portion being preferably provided within the fluid path or at the second end of the fluid path.

This will provide the same advantages as discussed above with respect to the second and third aspect.

The method may in particular be used for the manufacture of a transducer protector as described above with respect to the first, second and/or third aspect of the present invention.

The present invention is now described in further detail with respect to drawings and embodiments.

In the drawings, the figures show the following:
- Fig. 1: first embodiment of a transducer protector of the present invention in a side view,
- Fig. 2: the first embodiment in a perspective view when seen from the first side,
- Fig. 3: the first embodiment of a transducer protector, coupled to a first embodiment of a pressure transducer interface according to the present invention, in sectional view,
- Fig. 4: the first embodiment of a transducer protector connected to a second embodiment of a pressure transducer interface according to the present invention, in a sectional view,
- Fig. 5: the first embodiment of the transducer protector coupled to a third embodiment of a pressure transducer interface according to the present invention, in a sectional view,
- Fig. 6: a fourth embodiment of a pressure transducer interface in two perspective views,
- Fig. 7: the fourth embodiment of the pressure transducer interface with the first embodiment of the transducer protector connected thereto, in two perspective views,
- Fig. 8: a second embodiment of a transducer protector connected to a firth embodiment of a pressure transducer interface in a sectional view,
- Fig. 9: a third embodiment of a transducer protector coupled to a sixth embodiment of a pressure transducer interface in a sectional view,
- Fig. 10: a fourth embodiment of a transducer protector in a perspective view,
- Fig. 11: a fifth embodiment of a transducer protector in a perspective view,
- Fig. 12: a sixth embodiment of a transducer protector in a perspective view and
- Fig. 13: an embodiment of a tubing set comprising a component of an extracorporeal blood circuit and the inventive transducer protector.

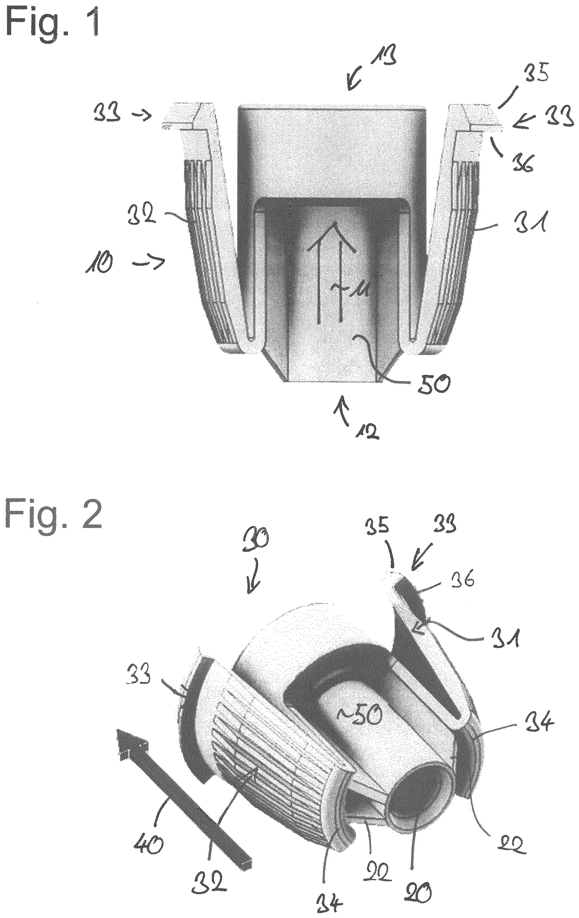

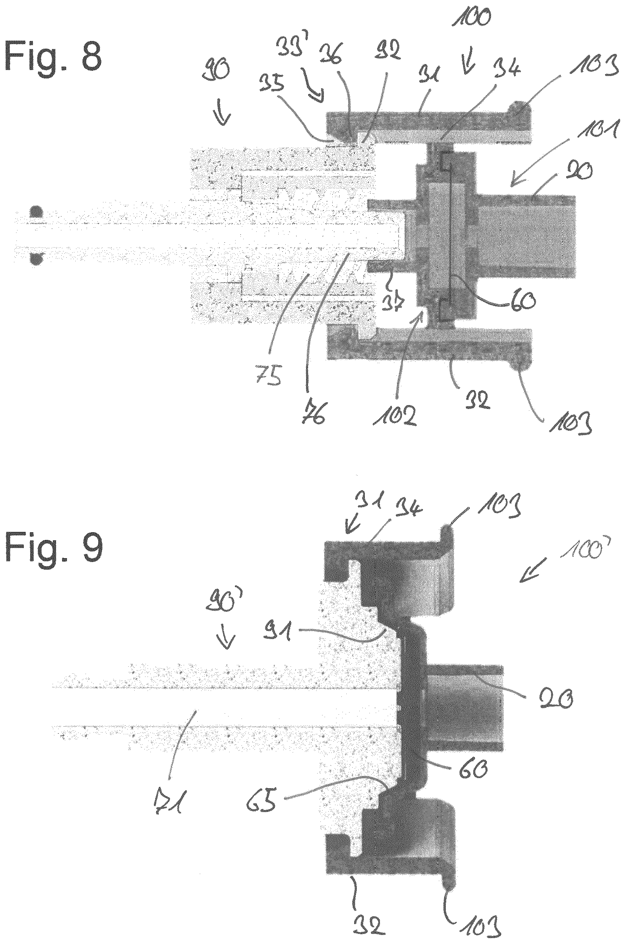

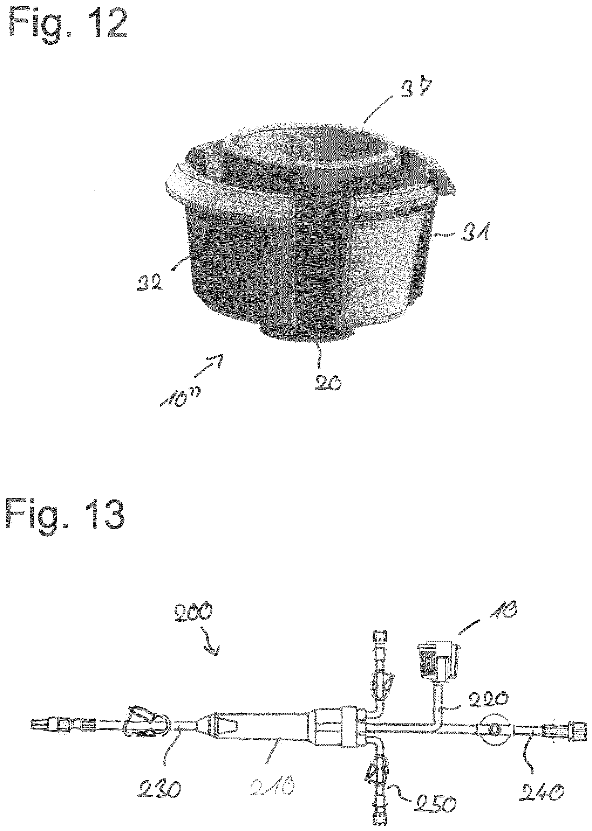

Fig. 1 - 5 and 7 show a first embodiment of a transducer protector according to the present invention. Further embodiments are shown in Fig. 8 to 12. Fig. 13 shows an embodiment of a tubing set comprising an extracorporeal blood circuit and the inventive transducer protector. If not otherwise stated, the features and the configuration of the transducer protector described in the following applies to all the embodiments.

Further, the first embodiment of a transducer protector, as well as most of the other embodiments, embody all the aspects of the present invention in combination. However, the features described with respect to the respective aspects could also be used independently from the other aspects.

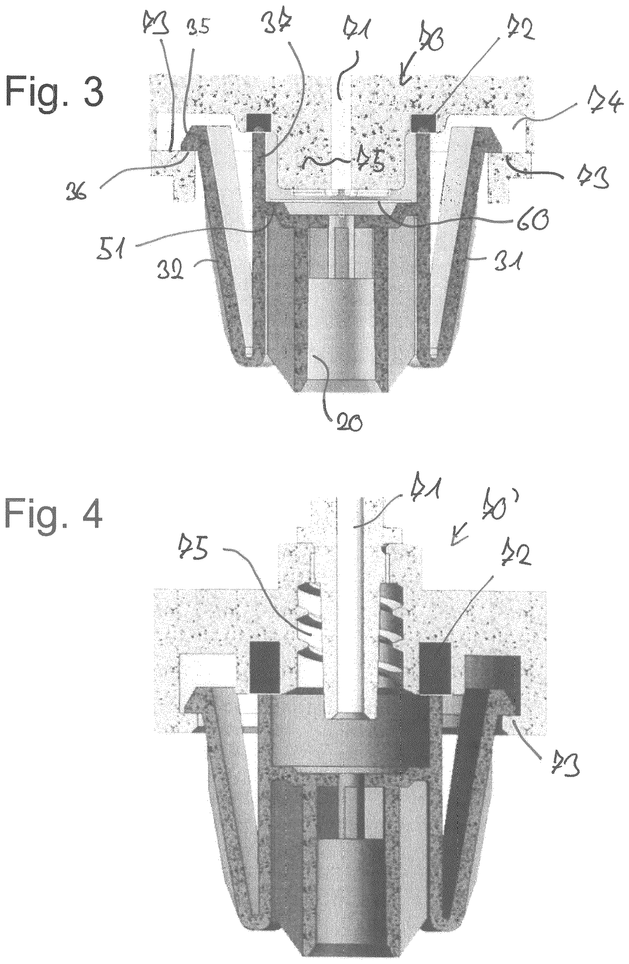

According to the present invention, the transducer protector 10 comprises a main body 50 comprising a fluid path 11 schematically shown by an arrow in Fig. 1. The fluid path 11 passes through the main body of the transducer protector from a first end 12 to a second end 13. Further, a hydrophobic gas-permeable membrane 60 shown in Fig. 3 is arranged in the fluid path 11 for separating the first end of the fluid path from the second end.

The hydrophobic gas-permeable membrane is in particular an air-permeable hydrophobic membrane. It therefore provides an air-permeable and therefore pressure-permeable connection through the connector, but is impermeable to liquids and therefore protects the second end 13 from blood entering through the first end 12.

The transducer protector 10 comprises a first connector 20 for fluidly coupling the first end 12 of the fluid path to a pipe of an extracorporeal circuit. In particular, as shown in Fig. 2 and 3, the first connector may be provided by a tubular element 20, into which the distal end of the pipe of the extracorporeal circuit may be inserted. The end of the pipe may be bonded to the first connector and in particular to the inside of the tubular element 20 by an adhesive.

The transducer protector further comprises a second connector for fluidly coupling the second end 13 of the fluid path to a pressure transducer interface, such as one of the pressure transducer interfaces shown in Fig. 3 - 9. In most of the embodiments, the second connector 30 comprises a second tubular element for sealingly connecting to a fluid path of the pressure transducer interface. In some embodiments, the second tubular element 37 has a larger diameter than the first tubular element 20 forming the first connector.

According to the first aspect of the present invention, the second connector 30 of the present invention is configured as a snap-fit connector. In the embodiment shown, the snap-fit connection between the second connector and the pressure transducer interface is engaged by an axial movement of the transducer protector, as indicated by arrow 40 in Fig. 2.

Therefore, for closing the snap-fit connection, no rotating movement is needed. In contrast to the prior art solutions using a Luer connection, fixation is ensured by a single axial movement. This provides an improved usability.

The snap-fit connection is provided by snap-fit elements 31 and 32 of the second connector 30. In the embodiments, the snap-fit elements are provided as snap-fit arms of the cantilever type, wherein at least a section of the snap-fit arm extends from a connection point 34 with the main body of the transducer protector towards the pressure transducer interface in a direction along the fluid path 11 of the pressure transducer.

In the embodiments shown, the snap-fit arms 31 and 32 form or comprise handling sections for the transducer protector, which are used for gripping the transducer protector and for operating the snap-fit connection, in particular for releasing the snap-fit connection by applying pressure to the handling sections. This provides an enlarged handling area and therefore an improved usability with respect to the prior art.

Further, due to the snap-fit connection, a proper connection is ensured by tactile and audible feedback. In particular, the snap-fit connection will provide a click sound and an abrupt jump in the counter pressure against an axial movement when the snap-fit connection is engaged.

The snap-fit arms 31 and 32 are provided with a snap-fit geometry 33 at their free end, which is configured for engaging with a counter element 73, 82 or 83 or 92 of the pressure transducer interface.

The snap-fit geometry 33 of each snap-fit arm in particular comprises a hook section 36, in particular provided by a step surface projecting away from a main outer or inner surface of the snap-fit arm, as well as a chamfer section 35, in particular in the form of a ramp, which forms the first contact with the counter element.

In particular, due to the contact of the chamfer section 35 with the counter element, the snap-fit arm will be deformed by an axial movement of the transducer protector to allow the hook section 36 to pass the counter element, in order to engage with a hook section of the counter element.

For this purpose, the chamfer section 35 is provided at the free end of the snap-fit arm in front of the hook section 36.

In one embodiment, the hook section 36 comprises a circular arc and has an annular shape. I.e., the width of the hook section is constant in radial direction.

In another embodiment of the present invention, the hook section 36 comprises a circular arc and has a crescent or sickle shape. I.e., the width of the hook section in radial direction is not constant but has its maximum at the middle of the arc.

In most of the embodiments shown, the main body of the transducer protector is provided by the two tubular sections 20 and 37 of the first and the second connector 20 and 30.

In all the embodiments shown, at least two snap-fit arms 31 and 32 are provided on opposite sides of the main body with respect to an axis provided by the fluid path.

In all the embodiments shown, the fluid path defines a single axis through the connector, with the first tubular element 20 and the second tubular element 37 being arranged co-linear with respect to each other.

Further, in all the embodiments but for the embodiment shown in Fig. 9, the snap-fit arms 31 and 32 extend from the connection point 34 with the main body along an outer side of the main body but separately from the main body and with a distance thereto towards the pressure transducer interface. In particular, they may extend from the connection point 34 with the main body along an outer side of a tubular element forming the second end of the fluid path but separately from the tubular element and with a distance thereto.

Thereby, the length of the snap-fit arms in an axial direction can be nearly as big as the length of the main body or the fluid path, thereby increasing the handling area available on the snap-fit arms.

In the first embodiment shown in Fig. 1 - 5 and 7, the transducer protector has the snap-fit geometry 33 arranged on an outer side of the snap-fit arms 31 and 32, and therefore engages with the counter elements of the pressure transducer interface on its outside.

For disengaging the snap-fit connection, the snap-fit arms 31 and 32 are therefore flexed with their free ends facing the pressure transducer interface towards each other and/or towards the main body.

Further, in these embodiments, the snap-fit arms 31 and 32 only extend in one axial direction from their connection point 34 with the main body, i.e. from the connection point 34 towards the pressure transducer interface. Further, the handling section of the snap-fit arms is provided between the connection point 34 and the free end facing the pressure transducer interface.

In the embodiments shown, the handling section is provided with ridges extending from the connection section 34 towards the free end. The ridges may alternatively be provided in a direction vertical to a direction from the connection point 34 and the free end.

In the embodiments shown, the snap-fit arms extend, when not deformed by external pressure, with an angle different from zero away from the central axis of the transducer protector from the connection point 34 towards their free end facing the pressure transducer interface. In particular, the angle may be between 2 ° and 25 °, in particular between 4 ° and 15 ° as an average over the extension of the snap-fit arm. This allows an easy manufacture by injection molding.

In the embodiment shown, the second tubular section 37 is connected to the first tubular section 20 by a step portion 51. Further, the inner diameter of the second tubular section 37 is larger than the outer diameter of the first tubular section 20.

Further, In the embodiment shown, the second tubular section 37 extends from the first end of the fluid path to the step portion 51 over its entire circumference, and extends beyond the step portion 51 further towards the first side in first parts of its circumference, with the connected end of the snap-fit arms 31 and 32 attached to the rear side of these first parts of the tubular element 37.

In the embodiment shown, the second tubular section 37 stops at the step portion in second parts of its circumference arranged between the first parts.

Further, the first parts of the second tubular element 37 are connected to the first tubular element 20 by webs 22 extending in an axial direction between the step portion 51 and the first end, and extending in a radial direction between the first tubular element 20 and the second tubular element 37.

According to a second aspect of the present invention, the main body comprising the fluid path of the first and third to sixth embodiment of the transducer protector is formed by injection molding as a single, integral part. In particular, the first and the second tubular elements may be formed by injection molding as a single, integral part.

Further, in the first and the third to sixth embodiment, all the entire body of the transducer protector including all the elements of the first and the second connector are formed by injection molding as a single, integral part.

As described in more detail in the following, in the second embodiment of the pressure transducer, the main body is formed by two shells connected to each other.

In the embodiments, according to the third aspect of the present invention, the hydrophobic membrane 60 is bonded to a step portion 51 of the transducer protector from the second side.

In all the embodiments but the embodiment shown in Fig. 8, the diameter of the fluid path on the second side is larger than the diameter of the membrane 60 to allow for a free insertion of the membrane and accessibility for a bonding tool through the second end of the fluid path during manufacture. The bonding of the hydrophobic membrane to the sealing layer may done by heatbonding, ultrasonic welding or glueing. The preferred method for bonding the membrane to the sealing layer is ultrasonic welding.

In particular, in the first embodiment as well as the fourth to sixth embodiment, the diameter of the second tubular element 37 is larger than the diameter of the membrane 60, with the membrane 60 being bonded to the step portion 51 connecting the first end of the tubular element 37 to the first tubular element 20.

Further, the step portion 51 has, at a position where the membrane is bonded thereto, a larger inner diameter than a connection hole in a bottom portion of the step portion connecting the inside of the second tubular element 37 with the inside of the first tubular element 20. In particular, the bottom of the step portion may be recessed with respect to a step surface to which the membrane is bonded, with the connection hole to the first tubular element being provided in the bottom wall.

In the embodiment, the step portion 51 also has, at a position where the membrane is bonded thereto, a larger inner diameter than the first tubular element 20.

Apart from a pressure transducer, the present invention also provides a novel pressure transducer interface configured for engaging with the inventive transducer protector. Different variants of such an interface 70, 70' and 70" are shown in Fig. 3-5.

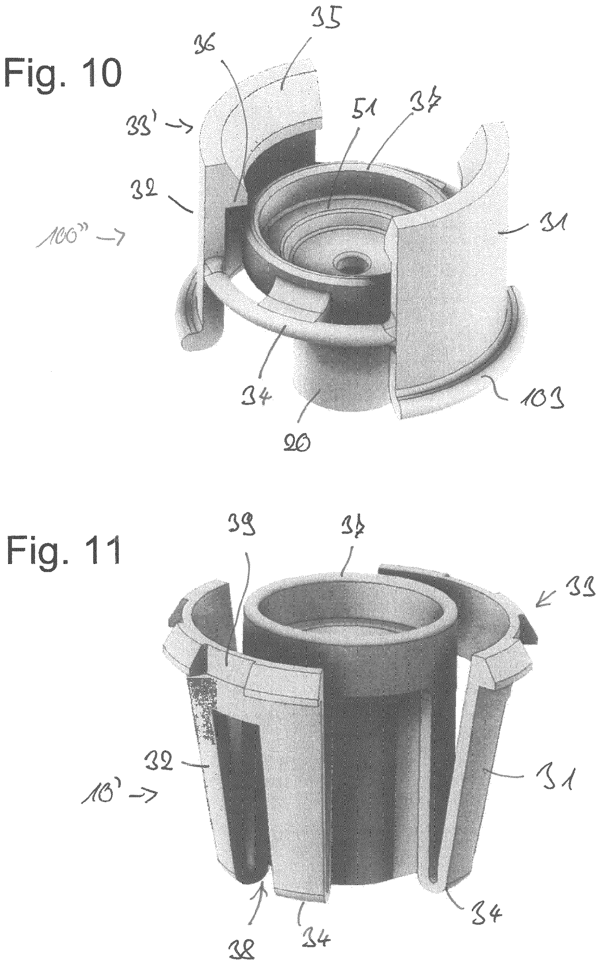

In these embodiments, the interface is configured to engage with the transducer protector of the first or fifth and sixth embodiment. The counter element 73 of the transducer interface therefore is provided with a snap-fit geometry and in particular a hook section on its inner side, engaging with the snap-fit geometry of the transducer protector provided on the outside of the snap-fit arms of the first embodiment shown in Fig. 1 - 5 and of the fifth and sixth embodiment shown in Fig. 11 and 12.

The sealing between the transducer protector and the pressure transducer interface is provided, in all the embodiments shown in Fig. 3 - 5, by a sealing element 72 arranged on the side of the pressure transducer interface. The sealing element 72 is configured to sealingly engage with the second tubular section 37 of the transducer protector. In particular, the sealing element 72 is provided by an O-ring made from an elastomeric material.

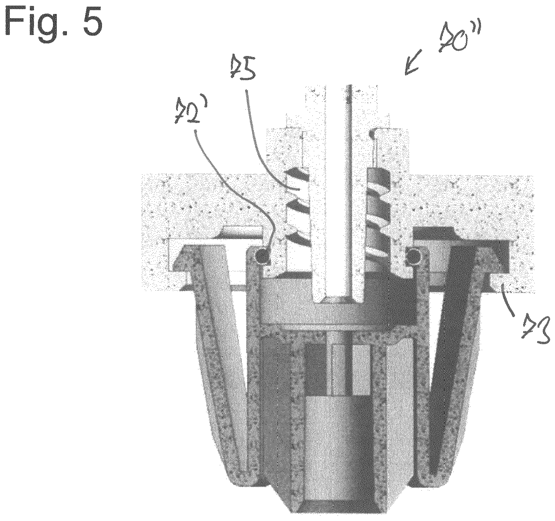

In the embodiments shown in Fig. 3 and 4, the sealing element 72 engages with a front surface of the tubular element 37. In Fig. 5, the sealing element 72' engages with an inner side surface of the tubular element 37. In an alternative configuration, it could also engage with an outer side surface of the tubular element 37.

In Fig. 3 and 4, the sealing element 72 has a rectangular cross-section and is provided in a groove facing the transducer protector.

In Fig. 5, the sealing element has a circular cross-section and is provided in a groove provided on the outside of a tubular section of the pressure transducer interface.

In the embodiments shown, the transducer protector of the present invention can be used with different kinds of machine interfaces, as shown in Fig. 3 - 5.

Fig. 3 shows a first embodiment, which is specifically designed to connect with the transducer protector of the present invention only. It comprises a projection 75 projecting into the tubular section 37 to reduce the air volume of the connector. Further, in this configuration, the membrane is better supported against accidental overpressure, because the front surface of the projection 75 is arranged close to the membrane 60 provided on the step portion 51 of the transducer protector as an abutment. A fluid path 71 connected to the pressure transducer extends through the projection 75.

The interfaces shown in Fig. 4 and 5 are configured to connect both to the transducer protector of the present invention, as well as to a prior art transducer protector comprising a standard Luer connection.

For this purpose, the interfaces shown in Fig. 4 and 5 comprise a threaded section 75 and a male Luer cone comprising the fluid path 71 connected to the pressure transducer, in order to connect to a female Luer connection with a screw connection.

In the embodiments shown in Fig. 3-5, the counter element of the snap-fit connection is provided within a recess 74. Such a recess may for example be provided as a recessed portion in a main surface of the interface.

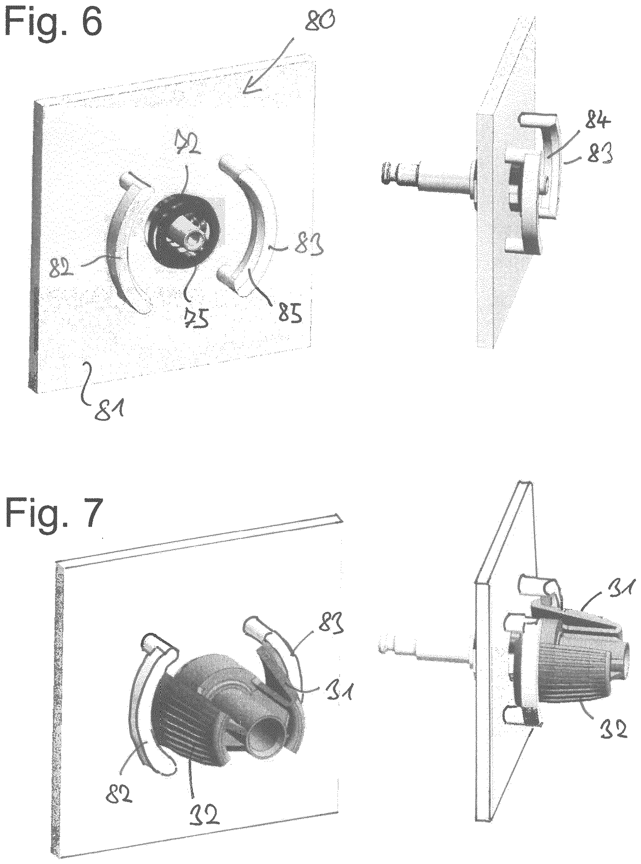

In contrast, Fig. 6 and 7 show an improved configuration of the interface that is easier to clean and disinfect, because the counter elements 82 and 83 for the snap-fit connection are provided by brackets projecting from the main surface 81 of the interface.

In particular, the brackets 82 and 83 are only connected at their ends to the main surface 81, and have a free bridge-like inner section that extends with a distance to the main surface 81 above the main surface 81. The hook section 84 of the counter element for engaging with a hook section 36 of the snap-fit arms 31, 32 is provided on this middle section of the brackets 82 and 84, as can be seen in Fig. 6 and 7 in the right-hand side drawing. Further, the brackets equally have a chamfer section 85 for contacting the chamfer section of the snap-fit arms of the transducer protector.

The brackets therefore can be accessed by a user from below to easily clean and disinfect around the fluid connection provided between the brackets.

The fluid connection as well as the sealing for the embodiment shown in Fig. 6 and 7 can be configured in any of the variants shown in Fig. 3 and 5. In the embodiment shown, the fluid connection is configured as shown in Fig. 5, i.e. with a threaded Luer connection 75 and an external O-ring 72. Alternatively, the fluid connection and the sealing could be configured as shown in Fig. 3 and 4, as well.

As particularly visible in Fig. 2 as well as in Fig. 6 and 7, the outer circumference of the snap-fit arms 32 and 31 is circular when seen in a cross-section in all the embodiments. Therefore, the snap-fit geometry is equally an arc section when seen in a section perpendicular to the axes of the transducer protector. This configuration provides a centering effect of the transducer protector when it is axially pressed in direction 40 towards the counter element on the machine interface side.

As shown in Fig. 6 and 7, the brackets are therefore formed by semi-circular elements. The recesses shown in Fig. 3 - 5 for the counter elements equally have a circular or semi-circular configuration.

Further variants of the transducer protector and the pressure transducer interface are now described with respect to Fig. 8 - 12. In the following, only those aspects in which these variants differ from the embodiments described so far are described in detail. Otherwise, the description of the embodiments provided above also refers to the embodiments shown in Fig. 8 - 12.

While the first embodiment of the transducer protector described so far has a snap-fit geometry on the outside of the snap-fit arms, the second to fourth embodiment shown in Fig. 8 - 10 comprises the snap-fit geometry on the inner side of the snap-fit arms and therefore engages with a counter snap-fit geometry arranged on an outer side of the counter element.

In particular, the snap-fit geometry 33' comprising the chamfer section 35 and the hook section 36 is provided on an inner side of the snap-fit arms 31 and 32, for engaging with a hook section 92 provided on the outside of a tubular element of the pressure transducer interface.

Further, the snap-fit arms 31 and 32 have a first section extending from the connection point 34 with the main body towards the first side of the transducer protector 100, 100' and 102', and a second section extending from the connection point 34 towards the second end and the pressure transducer interface. Thereby, by applying a pressure on the outer side of the first section, the first section of the snap-fit arms can be flexed to an inner side towards the main body, leading to movement of the second section comprising the snap-fit geometries 33' away from the main body, releasing the snap-fit connection. Therefore, in the embodiments shown in Fig. 8 - 10, the handling section 103 is provided on the first section between the connection 34 with the main body and the free end of the first section facing the first end. In particular, the handling section 103 is provided by a ring section projecting from the free end of the first section towards the outside.

The fourth embodiment shown in Fig. 10 is otherwise similar to the first embodiment shown in Fig. 1 - 5, with the main body being formed by the first tubular element 20 of the first connector and the second tubular element 37 of the second connector, the step section 51 arranged therebetween to which the membrane is bonded from the second side. The bonding of the hydrophobic membrane is done by heat bonding, ultrasonic welding or gluing. The preferred method for bonding the membrane to the sealing layer is ultrasonic welding.

In this embodiment, the two snap-fit arms 31 and 32 extend along the outer surface of the tubular section 37 with a gap being formed between the outer surface of the tubular section 37 and the inner surface of the snap-fit arms 31 and 32 over their entire axial length, in order to simplify the mold for injection molding the transducer protector. The two snap-fit arms 31 and 32 are connected to each other via a connection element extending between the two snap-fit arms and connected to the tubular section 37 at a position between the two snap-fit arms. In the embodiment, the connection element is ring-like.

The sealing and the fluid connection of this transducer protector can be configured as shown in Fig. 3-7, and only requires a different configuration of the counter element for the snap-fit geometry with a hook section provided on an outside rather than an inside of the counter element.

Both in the first and the fourth embodiment, as well as the fifth and the sixth embodiments, which will be described later on, the entire transducer protector is injection molded as an integral element, with the membrane being provided from the second side of the fluid path.

In contrast, in the embodiment shown in Fig. 8, the main body is made by two shells similar to the prior art solution, with the membrane 60 arranged between two shells at an enlarged middle section arranged between the first tubular section 20 of the first connector and the second tubular section 37 of the second connector, the two shells being welded to each other. In this embodiment, the snap-fit arms 31 and 32 are attached with their connection points 34 on the outer circumference of the enlarged middle section 102.

Further, the fluid connection between the second tubular section 37 and the interface is not provided by an O-ring, as in the other embodiments, but by a Luer connection between the tubular section 37 and the Luer cone 76 of the standard Luer connection, which is also provided with a standard screw section 75 for engaging with a standard Luer connector.

Therefore, the interface shown in Fig. 8 is compatible both with a prior art Luer connection of a transducer protector as well as the second embodiment of the inventive transducer protector using a snap-fit connection.

In contrast, in the embodiment shown in Fig. 9, the interface 90' is configured to be used only with an embodiment of a transducer protector according to the present invention.

In this embodiment also, an external snap-fit element is used, as already described with respect to Fig. 8 - 10. In this embodiment, the sealing is provided by an elastomeric component 65 which is part of the transducer protector, with the membrane being over-molded on the elastomeric component. In particular, the pressure transducer interface may comprise a conical section 91 engaging with a conical section provided at the second end of the fluid path, with the elastomeric material being provided as an elastomeric layer on the cone section of the transducer protector. In this embodiment, the connection 34 between the snap-fit arms is provided on an outer circumference of the cone section of the transducer protector, with the cone section being connected to the tubular section 20 by a step portion. The elastomeric material may in particular be a TPE. Therefore, in this embodiment, the membrane is bonded to the main body of the pressure transducer from the second side essentially at the second end of the fluid path.

In the embodiments shown in Fig. 8 and 9, the snap fit arms 31, 32 are connected to the main body of the transducer protector at a circumferential position of the main body between the snap fit arms 31, 32, as in the embodiment shown in Fig. 10.

Fig. 11 and 12 show two variants of the first embodiment of the transducer protector. Both embodiments have an internal snap-fit element as the first embodiment, and the same general configuration.

The embodiment shown in Fig. 11 has a reduced mass and material consumption than the first embodiment, with cutouts 38 provided in the snap-fit arms 31 and 32 extending from the first side towards the second side, thereby splitting the circumference of the snap-fit arms into several arm sections on the first side thereof. In the area of the cutouts 38, the second tubular section 37 equally ends already at the step section 51 and does not further extend towards the first side.

The distal ends of the remaining sections of the snap-fit arms are connected at the second side of the transducer protector by a partial ring section. The snap-fit geometry is provided, in the embodiment, only where the material of the snap-fit arms extends, while it is not provided in the circumferential portions where there are the cutouts 38.

In the embodiment shown in Fig. 12, two additional wings or snap-fit arms 39 are provided in between the snap-fit arms 31 and 32. The user however only has to press the snap-fit arm 32 and 32' to remove the transducer projector, and the additional wings 39 are included only to improve the alignment if the counter element of the pressure transducer interface is provided as a circular recess.

In the embodiment, a snap geometry is provided on the free end of the wings 39 for snapping into the counter element. However, the snap angle of the snap geometry of the wings 39 is not 90 degrees, such that they will disengage just due to the axial removal movement, and do not need to be pressed for disengaging the snap connection.

In contrast, the snap connection provided by the snap arms 31 is only disengaged, in all the embodiments, by applying a pressure on the snap-fit arms in a radial direction. In particular, the snap angle of the snap geometry 33 and in particular of the hook section 36 as well as the corresponding hook section on the counter element is, for the first and the second snap fit arm 31 and 32, essentially at 90 degrees.

In all the embodiments, due to the snap-fit connection, the present invention provides a decisively easier handling. Further, for those embodiments where the main body of the transducer protector is provided as a single piece to be manufactured by injection molding, or the membrane is bonded to the main body from the second side of the fluid path, the manufacture is simplified with respect to the two-shell-design of the prior art solution.

Fig. 13 shows a tubing set 200 comprising a transducer protector 10 connected by line 220 to a component of an extracorporeal blood tubing set. In the embodiment, the component to which the transducer protector 10 is connected by line 220 is a venous air trap chamber 210. Connected to chamber 210 are connections lines 230 and 240 for connection to a venous line and an arterial line, as well as further connection lines 250.

The end of line 220 is inserted and glued into connector 20 of the transducer protector 10. The distal ends of connection lines 230, 240 and 250 are provided with connection elements for providing a releasable connection to further lines or components of the extracorporeal blood circuit, such as Luer-lock connectors.

## Claims

1. A transducer protector for protecting a pressure transducer connected to an extracorporeal circuit, comprising:
a main body comprising a fluid path;
a first connector for fluidly coupling a first end of the fluid path to a pipe of the extracorporeal circuit;
a second connector for fluidly coupling a second end of the fluid path to a pressure transducer interface; and
a hydrophobic gas-permeable membrane arranged in the fluid path for separating the first end of the fluid path from the second end;
**characterized in that**
the second connector is a snap-fit connector.

2. The transducer protector according to claim 1, the second connector is configured to be snap-fit engaged with the pressure transducer interface with a single axial movement in the direction of the fluid path towards the second end,
wherein preferably,
the second connector comprises at least one snap-fit element of the cantilever type, wherein the snap-fit element preferably is a snap-fit arm connected to a main body of the transducer protector and having a free end comprising a snap-fit geometry for snap engaging a counter element of the pressure transducer interface, wherein further preferably, the snap-fit arm extends from a connection point where it is connected to the main body of the transducer protector in a direction substantially along of the fluid path of the transducer protector towards the pressure transducer interface.

3. The transducer protector according to one of the preceding claims, wherein the snap-fit connection provided by the second connector is a releasable connection, wherein preferably, the second connector is configured such that the snap-fit connection is releasable by flexing at least one snap-fit element by applying pressure to a handling section of the snap-fit element, wherein the snap-fit element is preferably configured to be releasable by a pressure acting on the handling section of the snap-fit element from an outer side in a direction towards the main body of the connector.

4. The transducer protector according to one of the preceding claims, wherein the second connector comprises at least two separate snap-fit elements, the snap-fit elements being preferably arranged on opposite sides of an axis defined by the fluid path at its second end, wherein further preferably, the snap-fit connection provided by the second connector is releasable by pressing the handling sections of the snap-fit elements from an outer side in a direction towards each other.

5. The transducer protector according to one of the preceding claims, wherein the second end of the fluid path is configured as a tubular element to be sealingly engaged with a fluid path of the pressure transducer interface, wherein preferably a snap-fit element or snap-fit elements of the second connector extend from a connection point with the main body of the transducer protector towards the pressure transducer interface on an outside and with a distance to the tubular element, the snap-fit element or snap-fit elements being deformable towards and/or away from the tubular element.

6. The transducer protector according to one of the preceding claims, wherein a snap-fit geometry of the second connector is configured to snap engage a counter element of the pressure transducer interface from an inner side, wherein preferably, a handling section of at least one snap-fit element of the second transducer is arranged between a connection point where the snap-fit element is connected to the main body of the transducer protector and a free end of the snap-fit element configured for connecting to the pressure transducer interface.

7. The transducer protector according to one of the preceding claims, wherein the second connector comprises at least one snap-fit arm extending in a direction along of the fluid path of the transducer protector towards the pressure transducer interface, wherein the length of the snap-fit arm in a direction defined by the fluid path is at least 50% of the length of the fluid path, preferably at least 65%, further preferably at least 80%, and/or a snap-fit element or a snap-fit geometry of the second connector has an essentially circular shape with respect to an axis defined by the fluid path at its second end.

8. A transducer protector for protecting a pressure transducer connected to an extracorporeal circuit, in particular a transducer protector according to one of the preceding claims, the transducer protector comprising:
a main body comprising a fluid path;
a first connector for fluidly coupling a first end of the fluid path to a pipe of the extracorporeal circuit;
a second connector for fluidly coupling a second end of the fluid path to a pressure transducer interface; and
a hydrophobic gas-permeable membrane arranged in the fluid path for separating the first end of the fluid path from the second end;
**characterized in that**
the transducer protector comprises an integrally molded part comprising the entire fluid path and preferably both the first and the second connector, and/or the hydrophobic gas-permeable membrane is bonded to a step portion of the transducer protector, the bonding area of the hydrophobic gas-permeable membrane being accessible from the second end of the fluid path, the step portion being preferably provided within the fluid path or at the second end of the fluid path.

9. The transducer protector according to one of the preceding claims, wherein the inner diameter and/or the cross-section of the fluid path between its second end and the hydrophobic gas-permeable membrane is larger than the outer diameter and/or outer shape of the hydrophobic gas-permeable membrane, and/or wherein the inner diameter and/or the inner cross-section of the step portion is larger than the inner diameter and/or the cross-section of the first connector and/or the pipe connected thereto.

10. A pressure transducer interface configured for connecting to the second connector of a transducer protector as defined by one of the preceding claims, the pressure transducer interface comprising a fluid path coupled to a pressure transducer and a snap-fit counter element for engaging with a snap-fit geometry of a snap-fit element of the second connector.

11. The pressure transducer interface according to claim 10,
wherein the snap-fit counter element is formed by a bracket having a middle section for engaging with the snap-fit geometry of the snap-fit element of the second connector freely extending between two connection sections where it is connected to a main surface of the pressure transducer interface, wherein preferably, the main surface is an essentially flat surface from which the bracket extends in a direction towards the transducer protector,
and/or
wherein the pressure transducer interface comprises, in addition to the snap-fit counter element, a threaded section configured for engaging with another type of transducer protector comprising a Luer connector.

12. A tubing system comprising a transducer protector according to one of claims 1 to 9 and at least one component of an extracorporeal blood circuit, wherein the component of the extracorporeal blood circuit is connected to the first connector via a tube, preferably glued to or inserted into a section of the first connector, wherein preferably the tube is a tube leading from a venous chamber of the extracorporeal blood circuit for conveying air across the hydrophobic gas-permeable membrane.

13. A blood treatment machine, in particular a dialysis machine, comprising a pressure transducer interface according to any one of claims 10 or 11 and/or a tubing system according to claim 12.

14. Use of a transducer protector according to one of claims 1 to 7 for connecting a pipe of an extracorporeal circuit to a pressure transducer by snap-fit connecting a snap-fit element of the second connector of the transducer protector to a snap-fit counter element of a pressure transducer interface, in particular of a pressure transducer interface of a blood treatment machine, in particular of a blood treatment machine according to claim 13.

15. A method for manufacturing a transducer protector for an extracorporeal circuit, in particular a transducer protector according to one of claims 1 to 9, the transducer protector comprising a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end;
the method comprising the step of:
- integrally molding the main body of the protector comprising the entire fluid path and preferably both the first and the second connector,
and/or
- bonding the hydrophobic gas-permeable membrane to a step portion of the transducer protector by accessing the bonding area of the hydrophobic gas-permeable membrane from and preferably through the second end of the fluid path, the step portion being preferably provided within the fluid path or at the second end of the fluid path.
